**Europäisches Patentamt**

**European Patent Office**

**Office européen des brevets**

(11) Veröffentlichungsnummer: **0 148 463**
**B1**

(12) # EUROPÄISCHE PATENTSCHRIFT

(45) Veröffentlichungstag der Patentschrift:
**05.10.88**

(51) Int. Cl.⁴: **G 01 N 21/47,** G 01 N 33/53

(21) Anmeldenummer: **84115626.8**

(22) Anmeldetag: **17.12.84**

(54) **Photometrisches Verfahren zur Konzentrationsbestimmung bei Reaktionen, die unter Bildung oder Verbrauch von Streuzentren verlaufen.**

(30) Priorität: **27.12.83 DE 3347162**

(43) Veröffentlichungstag der Anmeldung:
**17.07.85 Patentblatt 85/29**

(45) Bekanntmachung des Hinweises auf die Patenterteilung:
**05.10.88 Patentblatt 88/40**

(84) Benannte Vertragsstaaten:
**AT BE CH DE FR GB IT LI LU NL SE**

(56) Entgegenhaltungen:
**US - A - 4 157 871**
**US - A - 4 174 952**
**US - A - 4 204 837**
**US - A - 4 268 171**

**CLINICAL CHEMISTRY, Band 23, Nr. 8, 1977, Seiten 1456-1464, Baltimore, US; J.C. STERNBERG "A rate nephelometer for measuring specific proteins by immunoprecipitation reactions"**

(73) Patentinhaber: **BEHRINGWERKE Aktiengesellschaft, Postfach 1140, D-3550 Marburg 1 (DE)**

(72) Erfinder: **Metzmann, Erwin, Dr., Höhenweg 44, D-3550 Marburg 1 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Bestimmung der Konzentration eines Partners einer Reaktion mittels einer Lichtstreuungsmessung.

Das Phänomen der Lichtstreuung an Teilchen, die sich in einem homogenen Medium befinden, wird sowohl über die Messung der Streulichtintensität (Nephelometrie) als auch über die Messung des Intensitätsverlustes des durch das Medium tretenden Lichtstrahls (Turbidimetrie) zur Konzentationsbestimmung genutzt.

Viele chemische Reaktionen führen in mehreren Schritten zu Molekülaggregaten oder Makromolekülen, die sich in ihrem Streulichtverhalten sehr stark von den Ausgangsstoffen unterscheiden und deren Konzentration über diese Eigenschaft bestimmt werden kann.

Zum Beispiel treten bei Überschreitung des Löslichkeitsproduktes sehr viele Ionen zu Kristallen zusammen, die zunächst als Trübung erscheinen und schliesslich so gross werden, dass sie aus der flüssigen Phase sedimentieren.

Ein anderes Beispiel ist die immunchemische Reaktion zwischen einem löslichen Antigen und einem bivalenten Antikörper, die zu grossen und stark lichtstreuenden Molekülaggregaten führen kann.

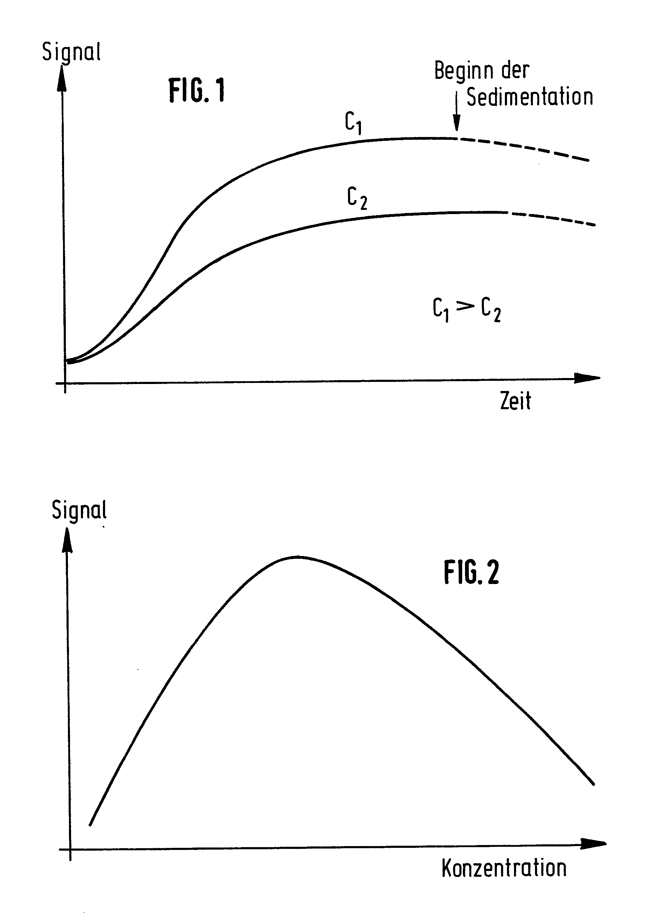

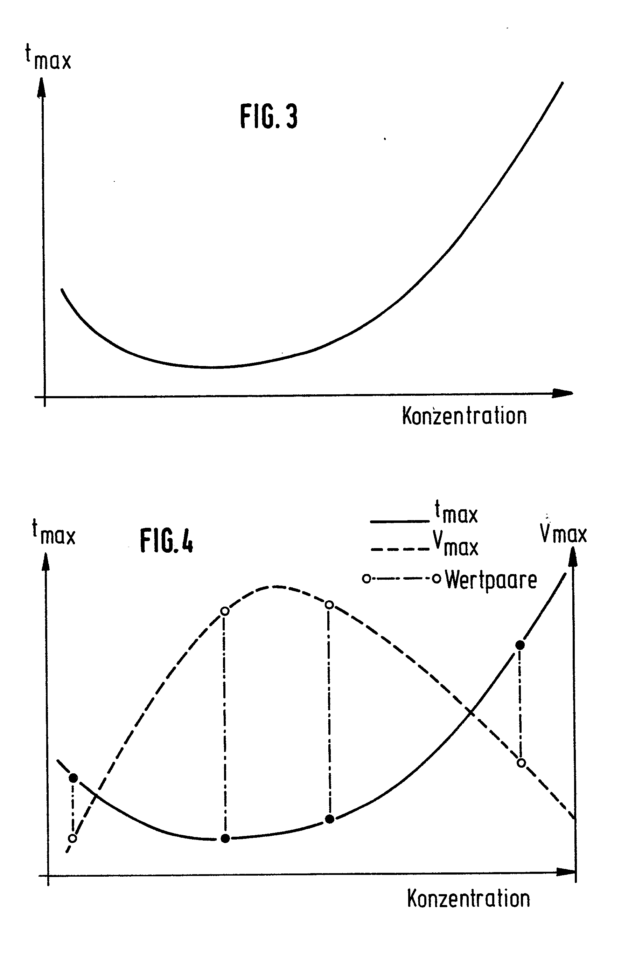

Der zeitliche Verlauf solcher Reaktionen entspricht sehr häufig dem allgemeinen kinetischen Verlauf aufeinanderfolgender Reaktionen 1. Ordnung, das heisst die Konzentrationskurve für die Bildung des Endproduktes zeigt einen Wendepunkt und somit tritt die maximale Reaktionsgeschwindigkeit erst im Laufe der Reaktion auf. Am Beispiel einer immunchemischen Reaktion ist dieser Verlauf für zwei Antigenkonzentrationen in Fig. 1 dargestellt.

Aus den Signal-Zeit-Kurven in Fig. 1 kann auf verschiedene Weise ein konzentrationsabhängiges Messsignal gewonnen werden (Tab. 1).

Tabelle 1:
Zeitliche Randbedingungen bei der Gewinnung von konzentrationsabhängigen Messsignalen (S = Signal, t = Zeit)

| Name | Prinzip |
|---|---|
| 1) Endpunktverfahren | Das Messsignal wird zu einem so späten Zeitpunkt ermittelt, bei dem es sich erfahrungsgemäss nicht mehr verändert, aber noch keine Präzipitation stattfindet. |
| 2) Kinetisches Verfahren «fixed-time Kinetik» | Das eigentliche Messsignal ist die Differenz zweier Signale, die zu verschiedenen, aber fest vorgegebenen Zeitpunkten ermittelt werden. |
| 3) Kinetisches Verfahren «rate determination» «peak rate method» | Die maximale Reaktionsgeschwindigkeit, d.h. die maximale Änderung des Signals pro Zeiteinheit, wird ermittelt; a) durch Messungen von delta S in hinreichend kurzen Intervallen von delta t und Bestimmung des grössten Quotienten delta S/delta t. b) elektronische Differenzierung dS/dt und Bestimmung des Maximums c) Konstruktion der Tangente an die Signal/Zeit-Kurve und Bestimmung der maximalen Steigung. |

Eine grosse Zahl von Analyten kann heute nach den in Tab. 1 aufgeführten Verfahren mit direkter oder indirekter Streulichtmessung quantifiziert werden.

Betrachtet man die Abhängigkeit eines geeigneten Messsignals (Tab. 1) von der Konzentration eines Reaktionspartners, z.B. des Antigens, während der andere Reaktionspartner mit konstanter Konzentration eingesetzt wird, so kann man zum Beispiel bei immunchemischen Reaktionen, den in Abb. 2 dargestellten Zusammenhang beobachten. Die überraschende Tatsache, dass das gleiche Messsignal sowohl auf eine niedrige als auch auf eine hohe Konzentation des Analyten zurückgeführt werden kann, führt zu einer Zweideutigkeit der Signal-Konzentration-Beziehung, die dem Fachmann als Antigenüberschuss-Phänomen oder Heidelbergerkurve bekannt ist.

Diese Zweideutigkeit ist prinzipiell überall dort beobachtbar, wo Komplexe unterschiedlicher Stöchiometrie möglich sind, je nach Überschuss des einen oder anderen Reaktionspartners, und sich diese Komplexe in ihrer Signaleigenschaft, zum Beispiel Streulicht, nicht unterscheiden.

Zum Beispiel:

$$\text{Mon}_x\text{CoMon}_{x+m} \quad \text{und} \quad \text{Mon}_{x+m}\text{CoMon}_x \qquad \text{wobei} \qquad \text{Mon} = \text{MonomerAg}_2$$
$$\text{CoMon} = \text{Co-Monomer}$$

$$\text{Ag}_2\,\text{Ak} \quad \text{und} \quad \text{AgAk}_2 \qquad \text{wobei} \qquad \text{Ag} = \text{Antigen}$$
$$\text{Ak} = \text{homologer Antikörper}$$

$$\text{Lec}_2\,\text{GP} \quad \text{und} \quad \text{Lec GP}_2 \qquad \text{wobei} \qquad \text{Lec} = \text{Lectin}$$
$$\text{GP} = \text{Glykoprotein}$$

$$(\text{Ag}_x\text{Cl}_{x+1})^- \quad \text{und} \quad (\text{Ag}_{x+1}\text{Cl}_x)^+ \qquad \text{wobei} \qquad \text{Ag} = \text{Silberion}$$
$$\text{Cl} = \text{Chloridion}$$

Führt man solche Reaktionen zum Zwecke der Konzentrationsbestimmung durch, so definiert und optimiert man das Verfahren für eine bestimmte Überschuss-Situation. In der Praxis führt dies dort zu Schwierigkeiten, wo der Analyt in einem sehr grossen Konzentrationsbereich vorliegen kann und der Konzentrationsüberschuss eines Reaktionsteilnehmers nicht sichergestellt werden kann.

Diese Situation spielt besonders eine Rolle bei der immunchemischen Bestimmung der Immunglobuline, deren Konzentration um den Faktor 1000 schwanken kann. Die Testbedingungen und die ökonomisch optimale Antikörperkonzentration zur Bestimmung der normalen und subnormalen Konzentration erlauben nicht die Erfassung einer um den Faktor 1000 erhöhten Konzentration.

Das folgende bezieht sich deshalb nur auf immunchemische Reaktionen, obwohl das Verfahren prinzipiell auf alle Reaktionen anwendbar ist, welche die oben erörterten Eigenschaften aufweisen und ein analoges kinetisches Verhalten zeigen.

In Bezug auf immunchemische Reaktionen sind eine Fülle von Verfahren und Ausführungsformen bisher vorgeschlagen worden, um zu erkennen, auf welchem Teil der Heidelbergerkurve man sich mit einem gegebenen Analysenansatz befindet.

Im folgenden seien einige Beispiele genannt:

1. Das älteste und sicherste Verfahren zur Erkennung eines Antigenüberschusses ist die Durchführung einer Doppelbestimmung mit zwei verschiedenen Probenverdünnungen. Bei Vorliegen eines Antigenüberschusses erhält man bei der grösseren Verdünnung ein höheres Signal und findet scheinbar einen höheren Proteingehalt als bei der konzentrierteren Probe. (H.E. Schultze u. G. Schwick; Prot. Biol. Fluids 5, 15–25 (1958)).

Verbesserte Ausführungsformen dieses Verfahrens sehen den weiteren Zusatz von Antikörpern in den Testansatz vor, nachdem die Reaktion ein gewisses Gleichgewicht erreicht hat. Beim Vorliegen eines Antigenüberschusses tritt dann eine Signalerhöhung ein (T.O. Tiffany et al., Clin. Chem. 20, 1055–1061 (1974)). Ebenso lässt sich ein Antigenüberschuss durch Nachdosieren mit Antigenmaterial bekannter Konzentration erkennen (J.C. Sternberg; Clin. Chem. 23, 1456–1464 (1977)).

2. Im speziellen Fall der continous-flow-Technik wird das Vorliegen eines Antigenüberschusses durch Auftreten eines Doppelpeaks bemerkt (R.F. Ritchie; Protides Biol. Fluids 21, 569 (1974)).

3. Durch Betrachtung der Reaktionskinetik nach graphischer Aufzeichnung des Reaktionsverlaufes kann man bei turbidimetrischer Messung zwischen Antikörperüberschuss und «mildem» Antigenüberschuss unterscheiden (I. Deverill; Protides Biol. Fluids 26, 697 (1979)) (P.J.J. Van Munster et al., Clin. Chim. Acta 76, 377–388 (1977)).

4. Durch Bestimmung der Reaktionsdauer bis zum Auftreten der maximalen Reaktionsgeschwindigkeit bei nephelometrischer Messung kann eine Grösse berechnet werden, die eine Diskriminierung zwischen Antigen- und Antikörperüberschuss erlaubt (DE-A-27 24 722 C2).

Die unter den Punkten 2 und 4 genannten Verfahren unterscheiden sich vorteilhaft von den anderen Techniken dadurch, dass nicht alle Proben auf das Vorliegen eines Antigenüberschusses überprüft werden müssen, sondern dass während des Messvorganges eine Information darüber erhalten wird, ob eine Prüfung auf Antigenüberschuss durchgeführt werden muss.

Verfahren 2 ist an eine bestimmte Technik gebunden und nicht allgemein für nephelometrische oder turbidimetrische Proteinbestimmungen anwendbar.

DE-A-27 24 722 C2 als Stand der Technik geht davon aus, dass eine Funktion der maximalen Reaktionsgeschwindigkeit existiert, die es erlaubt, mit Hilfe eines Schwellenwertes dieser Funktion zwischen Antigenüberschuss und Antikörperüberschuss zu diskriminieren (Anspruch 1). In Spalte 12, Zeile 8–21 dieser Patentschrift wird ausgeführt, dass als Diskriminatorfunktion die Zeit (T) dienen kann, die von der Reaktionsauslösung bis zum Auftreten des Maximalwertes (H) der Reaktionsgeschwindigkeit verstreicht. Diese Ausführungen werden jedoch in Spalte 21, Zeilen 55–68, stark eingeschränkt, dort heisst es, dass kein einzelner Zeitwert existiert, bei dem der Antikörperüberschuss-Kurventeil auf der einen Seite dieses Wertes und der Antigenüberschuss-Kurventeil auf der anderen Seite läge. Die Erfindung löst das Problem durch Koordinatentransformation und die Einführung neuer Variablen.

Das Verfahren ist weiterhin dadurch gekennzeichnet, dass bei Feststellung eines Antigenüberschuss-Zustandes die Messung nach entsprechender Verdünnung der Probe zur Erzielung eines Antikörperüberschuss-Zustandes wiederholt wird. Diese zusätzliche Messung einer Probe, die im Antigenüberschuss-Zustand gefunden wird, ist material- und zeitaufwendig.

Der Erfindung liegt deshalb als Aufgabe die Schaffung eines Verfahrens zugrunde, welches es erlaubt, ohne zusätzliche Messung den Gehalt einer Probe an einem Antigen oder Antikörper zu bestimmen, auch wenn sich diese im Antigenüberschuss-Zustand des jeweiligen Testansatzes befindet.

Überraschenderweise wurde nun gefunden, dass durch Messung der Zeit, die von der Reaktionsauslösung bis zum Auftreten des Maximalwertes der Reaktionsgeschwindigkeit verstreicht, bei einem kinetischen Verfahren, d.h. bei Bestimmung der maximalen Reaktionsgeschwindigkeit als Funktion der Konzentration, auf beiden Seiten der Heidelbergerkurve quantitativ gemessen werden kann. Eine Unterscheidung zwischen Antikörperüberschuss und Antigenüberschuss ist nicht mehr notwendig.

Gegenstand der Erfindung ist deshalb ein Verfahren zur photometrischen Bestimmung der Konzentration eines Reaktanten einer Reaktion, die unter Bildung oder Verbrauch von Streuzentren im Sinne eines Übergangs von der Rayleigh- zur Mie-Streuung verläuft, bei welchem die maximale Reaktionsgeschwindigkeit $V_{max}$ und die Zeit vom Reaktionsbeginn bis zum Auftreten der maximalen Reaktionsgeschwindigkeit $t_{max}$ bestimmt werden und zur eindeutigen Bestimmung der Konzentration der funktionelle Zusammenhang zwischen Konzentration, $V_{max}$ und $t_{max}$ mit einem Standardpräparat empirisch ermittelt und $V_{max}$ und $t_{max}$ an der Probe gemessen werden, dadurch gekennzeichnet, dass von zwei möglichen Konzentrationen mit Hilfe des 2. Reaktionsparameters, $V_{max}$ oder $t_{max}$, die richtige Konzentration ausgewählt wird.

Die Beziehung zwischen Konzentration und $V_{max}$ und $t_{max}$ kann beispielsweise in einer Tabelle, einer Matrix oder einer oder mehreren mathematischen Funktionen wiedergegeben sein.

Es ist bekannt, dass Reaktionsgeschwindigkeit und Signalintensität von Antigen-Antikörper-Reaktionen nach Ionenstärke, Ionenart und Polymerzusatz des Reaktionsmediums in einem weiten Bereich beeinflusst werden können. Die folgenden Ausführungen beschränken sich deshalb nicht auf die in den Beispielen aufgeführten Bedingungen sondern gelten für Präzipitatsreaktionen, die sich im oben genannten Sinne beeinflussen lassen.

Bestimmt man die maximale Reaktionsgeschwindigkeit ($V_{max}$) in Abhängigkeit von der Konzentration, so erhält man den in Fig. 2 dargestellten Zusammenhang. Bestimmt man gleichzeitig mit $V_{max}$ die Zeit, die von Beginn der Reaktion bis zum Erreichen von $V_{max}$ verstreicht ($t_{max}$), so ergibt sich der in Fig. 3 dargestellte Zusammenhang. Jede der beiden Signal-Konzentrationskurven zeigt das Antigenüberschuss-Phänomen; bestimmten Signalen können zwei Konzentrationen zugeordnet werden.

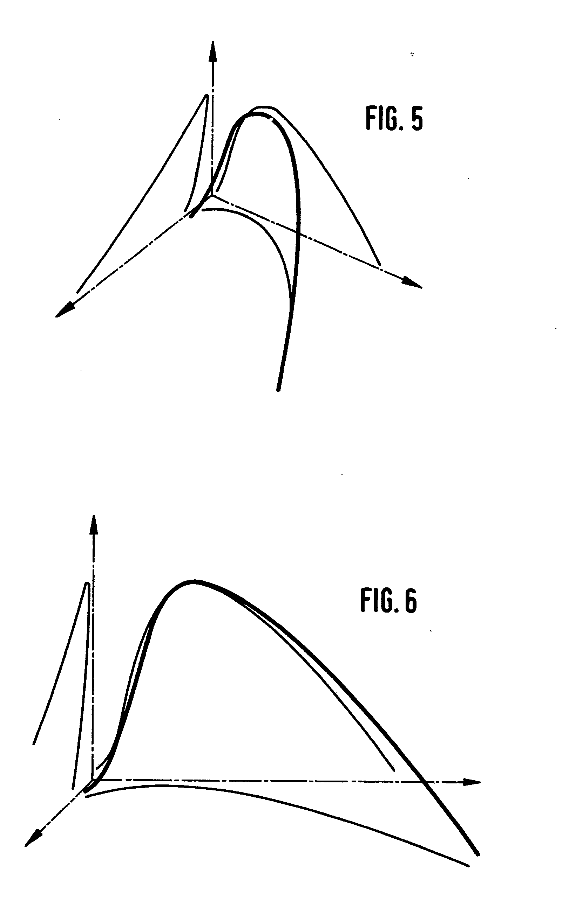

Projeziert man die beiden Funktionen in eine Abbildung (Fig. 4), so erkennt man, dass kein Wertepaar ($V_{max}$, $t_{max}$) existiert, das zwei verschiedenen Konzentrationen zugeordnet werden kann.

Die maximale Reaktionsgeschwindigkeit ($V_{max}$) und die Zeit bis zum Erreichen der maximalen Reaktionsgeschwindigkeit ($t_{max}$) sind offensichtlich voneinander unabhängige Variable der Konzentration, und die Heidelbergerkurve ist eine dreidimensionale Kurve (Fig. 5), die bei geeigneter Reaktionsführung keine Doppeldeutigkeit im Hinblick auf die Konzentration zeigt.

Messverfahren, bei denen nur mit einer zweidimensionalen Projektion dieser Kurve gearbeitet wird, z.B. Messung von $V_{max}$ als Funktion der Konzentration (Fig. 6), zeigen immer eine Doppeldeutigkeit im Hinblick auf die Konzentration.

Bei der praktischen Auswertung der gefundenen Zusammenhänge ist zu beachten, dass in der Nähe des Minimums oder des Maximums einer Signal-Konzentrations-Kurve die Konzentration nur mit einer verminderten Präzision bestimmt werden kann, da relativ geringe Signaldifferenzen einer grossen Konzentrationsdifferenz entsprechen. Bei dem neuen Verfahren der Bestimmung zweier unabhängiger Messsignale wird die Präzision besonders dann verbessert, wenn das Minimum der Funktion Konz. $= f(t_{max})$ möglichst weit von dem Maximum der Funktion Konz. $= f(V_{max})$ entfernt ist.

Arbeitsweise und Reaktionsbedingungen des neuen Verfahrens sollen in den folgenden Beispielen gezeigt werden.

Beispiel 1
Bestimmung des Maximum-Bereiches einer Kalibrierkurve für IgG

Photometer: DU-8 mit Zusatzteil für nephelometrische Messungen (Beckman Instruments)

Reaktionsmedium: 0.02 mol/l Kaliumphosphatpuffer pH 7.3 mit 40 g/l Polyäthylenglycol 6000 (Serva), Heidelberg, Bundesrepublik Deutschland und 64 g/l Natriumbromid

Probenverdünnungsmedium: Physiologische NaCl-Lösung

Antiserum: LN-Antiserum gegen Human IgG/kappa-Kette (vom Kaninchen) (Behringwerke AG, OSAS 14), 1:31 mit Reaktionsmedium verdünnt

Standard: T-Protein-Standard-Serum (human), (Behringwerke AG, OSKT 06)

Testansatz: 20 µl Proben-/Standardverdünnung werden mit 500 µl Antiserumverdünnung versetzt und sofort im Photometer unter Verwendung des Kinetik II-Programmes maximal 3 min. bei einer Wellenlänge von 334 nm gemessen.

a) Turbidimetrische Messung

| Konzentration der Standard- verdünnung in mg/dl IgG | Maximale Reak- tionsgeschwin- digkeit ($V_{max}$ in mE/min) | Zeit von Reak- tionsbeginn bis zum Auftreten des Maximums ($t_{max}$ in sec.) |
|---|---|---|
| 110.4 | 425 | 38 |
| 92 | 466 | 30 |
| 78.9 | 542 | 24 |
| 73.6 | 569 | 20 |
| 69 | 583 | 18 |
| 64.9 | 576 | 16 |
| 61.3 | 567 | 16 |
| 55.2 | 548 | 14 |
| 22.1 | 219 | 20 |
| 11.0 | 75 | 30 |

b) Nephelometrische Messung

| Konzentration der Standard- verdünnung in mg/dl IgG | Maximale Reak- tionsgeschwin- digkeit ($V_{max}$ in mE/min) | Zeit vom Reak- tionsbeginn bis zum Auftreten des Maximums ($t_{max}$ in sec.) |
|---|---|---|
| 110.4 | 1410 | 36 |
| 92 | 1490 | 30 |
| 78.9 | 1710 | 22 |
| 73.6 | 1730 | 22 |
| 69 | 1780 | 20 |
| 64.9 | 1800 | 20 |
| 61.3 | 1810 | 20 |
| 55.2 | 1720 | 18 |
| 22.1 | 706 | 24 |
| 11.0 | 325 | 42 |

Beispiel 1 zeigt, dass sowohl bei turbidimetrischer als auch bei nephelometrischer Messung Konzentrationen im und zu beiden Seiten des Maximums der Heidelbergerkurve eindeutig bestimmt werden können. Zur Ermittlung einer unbekannten Probenkonzentration wird nach Messung von $V_{max}$ und $t_{max}$ zwischen den tabellierten Werten des Standards interpoliert. Der Messbereich wird durch geeignete Verdünnung der Proben festgelegt, in Beispiel 1 bei 1:51 Verdünnung der Probe von 560 bis 5600 mg/dl IgG, und kann so gewählt werden, dass der Teilbereich mit der geringeren Präzision nicht in den klinisch relevanten Entscheidungsbereich des entsprechenden Parameters fällt.

Beispiel 2
Aufnahme einer Kalibrierkurve für IgM (turbidimetrisch)

Photometer: DU-8 (Beckman Instruments)

Reaktionsmedium: 0.15 m NaCl-Phosphatpuffer pH 7.2 mit 39 g/l PEG 6000 (Serva) und 8 g/l NaCl

Probenverdünnungsmedium: Physiologische NaCl-Lösung

Antiserum: LN-Antiserum gegen Human IgM/μ-Kette (vom Kaninchen) (Behringwerke AG, OSAT 14), 1:11 mit Reaktionsmedium verdünnt

Standard: T-Protein-Standard-Serum (human), (Behringwerke AG, OSKT 06)

Testansatz: 200 μl Proben-/Standardverdünnung werden mit 500 μl Antiserumverdünnung versetzt und sofort im Photometer unter Verwendung des Kinetik II-Programmes maximal 3 min. bei einer Wellenlänge von 312 nm gemessen

| Konz.Stand. verdünnung mg/dl IgM | Konz. Probe 1:21-Verdünn. in mg/dl IgM | Max. Reakt. geschwind. ($V_{max}$ in mE/min) | Zeit v. Reakt. beginn bis Maximum ($t_{max}$ in sec.) |
|---|---|---|---|
| 110 | 2310 | 371 | 34 |
| 74 | 1540 | 508 | 28 |
| 55 | 1155 | 591 | 18 |
| 44 | 924 | 639 | 14 |
| 37 | 770 | 639 | 12 |
| 31 | 660 | 620 | 12 |
| 28 | 578 | 611 | 10 |
| 24 | 513 | 598 | 10 |
| 22 | 462 | 556 | 10 |
| 20 | 420 | 508 | 10 |
| 18 | 385 | 488 | 10 |
| 17 | 355 | 467 | 10 |
| 16 | 330 | 460 | 10 |
| 15 | 308 | 419 | 10 |
| 14 | 290 | 405 | 10 |
| 13 | 272 | 378 | 10 |
| 12 | 257 | 368 | 10 |
| 11 | 231 | 330 | 10 |
| 5.5 | 116 | 158 | 10 |
| 2.8 | 58 | 69 | 10 |
| 1.4 | 29 | 27 | 12 |

Aus Beispiel 2 lässt sich entnehmen, dass bei der gewählten Probenverdünnung eine Konzentration zwischen 30 und 600 mg/dl IgM mit einer besseren Präzision gemessen werden kann als zwischen 600 und 900 mg/dl. Bei einem Normalbereich zwischen 70 und 300 mg/dl IgM im Serum legen jedoch Werte oberhalb von 600 mg/dl stets den Verdacht auf eine monoklonale Gammopathie nahe und erfordern, unabhängig von der Präzision des Wertes, weitere Untersuchungen.

## Patentansprüche

1. Verfahren zur photometrischen Bestimmung der Konzentration eines Reaktanten einer Reaktion, die unter Bildung oder Verbrauch von Streuzentren im Sinne eines Übergangs von der Rayleigh- zur Mie-Streuung verläuft, bei welchem die maximale Reaktionsgeschwindigkeit $V_{max}$ und die Zeit vom Reaktionsbeginn bis zum Auftreten der maximalen Reaktionsgeschwindigkeit $t_{max}$ bestimmt werden, zur eindeutigen Bestimmung der Konzentration der funktionelle Zusammenhang zwischen Konzentration, $V_{max}$ und $t_{max}$ mit einem Standardpräparat empirisch ermittelt und $V_{max}$ und $t_{max}$ an der Probe gemessen werden, dadurch gekennzeichnet, dass von zwei möglichen Konzentrationen mit Hilfe des 2. Reaktionsparameters, also $V_{max}$ oder $t_{max}$, die richtige Konzentration ausgewählt wird.

2. Verfahren nach Anspruch 1, dadurch gekennzeichnet, dass die Reaktion eine immunchemische Reaktion ist.

3. Verfahren nach Anspruch 1, dadurch gekennzeichnet, dass die photometrische Bestimmung mittels einer Durchlicht-Messung ausgeführt wird.

4. Verfahren nach Anspruch 1, dadurch gekennzeichnet, dass die photometrische Bestimmung mit Strahlung einer Wellenlänge von 240 bis 700 nm, vorzugsweise jedoch von 300 bis 380 nm, ausgeführt wird.

5. Verfahren nach Anspruch 1, dadurch gekennzeichnet, dass die photometrische Bestimmung mittels einer Streulicht-Messung ausgeführt wird.

## Claims

1. A method for the photometric determination of the concentration of a reactant in a reaction in which scattering centers are formed or consumed in the sense of a transition from Rayleigh to Mie scattering where the peak rate of reaction $V_{max}$ and the time from the start of the reaction to the appearance of the peak rate of reaction $t_{max}$ are determined and to obtain a single value for the concentration the functional relationship between concentration, $V_{max}$ and $t_{max}$ is found empirically using a standard preparation, and $V_{max}$ and $t_{max}$ are measured on the sample, which comprises choosing between the two possible concentration values on the basis of the 2nd reaction parameter, namely $V_{max}$ or $t_{max}$.

2. The method as claimed in claim 1, wherein the reaction is immunochemical reaction.

3. The method as claimed in claim 1, wherein the photometric determination is carried out by means of a transmitted light measurement.

4. The method as claimed in claim 1, wherein the photometric determination is carried out with radiation at a wavelength from 240 to 700 nm, preferably from 300 to 380 nm.

5. The method as claimed in claim 1, wherein the photometric determination is carried out by means of a scattered light measurement.

## Revendications

1. Procédé de détermination photométrique de la concentration d'une substance réagissant dans une réaction qui se déroule avec formation ou consommation de centres de dispersion au sens d'un passage de la dispersion de Rayleigh à la dispersion de Mie, dans lequel la vitesse de réaction maximale $V_{max}$ et le temps s'écoulant depuis le début de la réaction jusqu'à l'apparition de la vitesse de réaction maximale $t_{max}$ sont déterminées, les relations fonctionnelles entre concentrations, $V_{max}$ et $t_{max}$ sont déterminées empiriquement avec une préparation étalon pour détermination sans ambiguïté de la concentration, et $V_{max}$ et $t_{max}$ sont mesurés sur l'échantillon, caractérisé en ce qu'on choisit entre deux concentrations possibles à l'aide du deuxième paramètre réactionnel, c'est-à-dire $V_{max}$ ou $t_{max}$, la concentration correcte.

2. Procédé suivant la revendication 1, caractérisé en ce que la réaction est une réaction immunochimique.

3. Procédé suivant la revendication 1, caractérisé en ce que la détermination photographique est effectuée au moyen d'une mesure en lumière transmise.

4. Procédé suivant la revendication 1, caractérisé en ce que la détermination photographique est effectuée avec un rayonnement d'une longue d'ondes de 240 à 700 nm, mais de préférence de 300 à 380 nm.

5. Procédé suivant la revendication 1, caractérisé en ce que la détermination photographique est effectuée au moyen d'une mesure en lumière dispersée.

FIG. 1

Signal

$C_1$

Beginn der Sedimentation

$C_2$

$C_1 > C_2$

Zeit

FIG. 2

Signal

Konzentration

FIG. 3

FIG. 4

FIG. 5

FIG. 6